**Europäisches Patentamt**

**European Patent Office**

**Office européen des brevets**

(11) Numéro de publication: **0 198 759**
**B1**

(12) **FASCICULE DE BREVET EUROPÉEN**

(45) Date de publication du fascicule du brevet: **05.09.90**

(51) Int. Cl.⁵: **G 01 N 21/47, A 61 B 5/103**

(21) Numéro de dépôt: **86400698.6**

(22) Date de dépôt: **01.04.86**

(54) Appareil de mesure de la brillance de la peau.

(30) Priorité: **09.04.85 FR 8505331**

(43) Date de publication de la demande:
**22.10.86 Bulletin 86/43**

(45) Mention de la délivrance du brevet:
**05.09.90 Bulletin 90/36**

(84) Etats contractants désignés:
**AT BE CH DE FR GB IT LI LU NL SE**

(56) Documents cités:
**GB-A-2 009 922**
**GB-A-2 013 334**
**GB-A-2 022 244**

**PHYSICS IN MEDICINE AND BIOLOGY, vol. 25, no. 24, juillet 1980, pages 695-709, The Institute of Physics, Bristol, GB; J.B. DAWSON et al.: "A theoretical and experimental study of light absorption and scattering by in vivo skin"**

**PATENT ABSTRACTS OF JAPAN, vol. 5, no. 138 (P-78)810r, 2 septembre 1981; & JP-A-56 73 336 (RICOH K.K.) 18-06-1981**

(73) Titulaire: **SANOFI**
**40, Avenue George V**
**F-75008 Paris (FR)**

(72) Inventeur: **James, Yannick Christian**
**3, Justice Mauve**
**F-95000 Cergy (FR)**
Inventeur: **Comment, Alain**
**4, allée du Morvan**
**F-38130 Echirolle (FR)**
Inventeur: **Etienne, Jeanine, épouse Arnaud-Battandier**
**3, square des Feuillants**
**F-78150 Le Chesnay (FR)**
Inventeur: **Crisafulli, Emilio**
**Viale San Gimignano 12**
**I-20146 Milan (IT)**

(74) Mandataire: **Joly, Jean-Jacques**
**CABINET BEAU DE LOMENIE 55, rue d'Amsterdam**
**F-75008 Paris (FR)**

Courier Press, Leamington Spa, England.

EP 0 198 759 B1

# EP 0 198 759 B1

**Description**

La présente invention concerne un appareil de mesure de la brillance de la peau. Elle découle d'un projet de fin d'études à l'Ecole Nationale Supérieure de l'Electronique et de ses Applications. L'état de la technique est décrit dans GB—A—2009922 et Physics In Medicine And Biology, vol. 25, No. 24 Juillet 1980, pages 695—709.

La mesure de la brillance de la peau trouve des applications en pathologie et en cosmétologie. En particulier, la brillance de la peau peut être associée à d'autres paramètres tels que le taux de secrétion de sébum. La mesure de brillance trouve alors une utilité pour l'étude de la séborrhée. Elle peut présenter de l'intérêt pour l'étude d'autres maladies de la peau, telles que le lichen, SSM... En cosmétologie, l'invention est applicable à la mesure de l'effet de produits dits "anti-brillance" pour peaux grasses, afin notamment de pouvoir réaliser des tests objectifs d'efficacité. Une autre application de l'invention serait la classification de différents types de peau.

Il existe déjà différents procédés et dispositifs destinés à la mesure de brillance de surface, notamment dans le cadre de l'industrie des peintures et vernis pour déterminer le voile de brillance de surfaces revêtues ou enduites. Il a aussi été proposé d'utiliser la measure de brillance pour l'évaluation du fini de surface.

Ces procédés et dispositifs connus à usage industriel ne sont pas applicables à la measure de brillance de la peau.

Un premier problème à résoudre dans le cadre de cette application particulière est celui de l'influence de la couleur. En effet, avec les appareils connus qui effectuent simplement une mesure de réflexion spéculaire, les résultats obtenus pour différentes surfaces ne sont comparables entre eux que si ces surfaces ont la même couleur.

Pour s'affranchir de l'influence de la couleur, il a été proposé de substituer à la measure absolue de réflexion spéculaire une mesure relative entre réflexion spéculaire et réflexion diffuse. Toutefois, les appareils connus mettant en oeuvre cette mesure relative restent inappropriés à la mesure de la brillance de la peau.

En effet, les appareils à usage industriel comportent en général des optiques à lentilles de concentration qui requièrent un positionnement précis de l'appareil de mesure par rapport à la surface dont la brillance est à mesurer. Il est alors nécessaire que cette surface soit plane et que l'aire de mesure soit en général de dimensions relativement importantes.

Or, dans le cas de la peau, l'aire de mesure doit être relativement restreinte, pour conserver les caractéristiques uniformes dans cette zone et faire une mesure sur une surface aussi plane que possible, sans altérer les caractéristiques à mesurer par un aplatissement de la peau.

Il est de plus nécessaire de disposer d'un appareil de mesure qui soit facile à manipuler et ne demande pas une précision élevée de positionnement par rapport à la peau.

L'invention a donc pour but de fournir un appareil de mesure de brillance spécifiquement adapté à la mesure de brillance de la peau.

Ce but est atteint grâce à un appareil comportant, conformément à l'invention:

— une sonde comprenant un boîtier dont une face destinée à être amenée au contact de la peau présentant une ouverture,

caractérisé par une liaison souple à fibres optiques comprenant au moins trois conducteurs optiques qui, à une première extrémité, sont fixés dans le boîtier de la sonde en étant dirigés vers l'ouverture de celle-ci, un premier et un deuxième conducteurs ayant leurs extrémités orientées suivant une première et une deuxième directions symétriques l'une de l'autre par rapport à l'axe de l'ouverture, et un troisième conducteur ayant son extrémité orientée suivant une direction autre que ladite seconde direction.

— un dispositif de mesure comprenant: un dispositif d'émission de lumière couplé optiquement à l'autre extrémité du premier conducteur, un dispositif de réception de lumière couplé optiquement à l'autre extrémité du deuxième conducteur pour fournir un premier signal représentatif de la réflexion spéculaire, et à l'autre extrémité du troisième conducteur pour fournir un second signal représentatif d'une partie de la réflexion diffuse, et des moyens de traitement connectés aux dispositifs d'émission et de réception de lumière, munis de moyens de correction pour s'affranchir de variations de l'intensité de la lumière émise et de l'influence de la lumière ambiante, et destinés à fournir un signal relatif de brillance à partir des valeurs mesurées de réflexion spéculaire et de réflexion diffuse, et,

— un dispositif d'affichage recevant le signal de brillance pour indiquer l'amplitude de ce signal.

La structure de l'appareil de mesure conforme à l'invention, telle que définie ci-dessus, avec une sonde reliée à un dispositif de mesure au moyen d'une liaison souple à fibres optiques, apporte plusieurs avantages.

L'utilisation de fibres optiques ayant leurs extrémités fixées à l'intérieur du boîtier de sonde dans une disposition relative figée permet une miniaturisation de la sonde. Il en résulte la possibilité d'effectuer des mesures sur des surfaces réduites, notamment des surfaces inférieures à 1 cm$^2$, par exemple de 10 à 50 mm$^2$. Il en résulte aussi une utilisation très facile de l'appareil puisque la sonde est de dimensions réduites et est reliée au reste de l'appareil par une liaison souple. Cette facilité d'emploi est encore accrûe du fait que, contrairement aux systèmes utilisant des optiques avec lentilles de concentration de faisceau qui demandent une grande précision de positionnement de l'appareil sur une surface plane, l'appareil

2

EP 0 198 759 B1

conforme à l'invention peut tolérer quelques degrés d'écart de position relative entre la sonde et la surface de la peau.

La compensation de variations d'intensité de la lumière émise et de l'influence de la lumière ambiante permet d'obtenir une mesure avec une bonne précision sans qu'il soit nécessaire de suivre des conditions opératoires très strictes.

Les moyens de corrections destinés à compenser des variations d'intensité de lumière émise peuvent être réalisés sous forme d'un circuit de régulation d'une source de lumière du dispositif d'émission, utilisant des moyens d'asservissement.

En variante, des moyens peuvent être prévus pour mesurer le flux lumineux produit par le dispositif d'émission afin de compenser les variations éventuelles de ce flux directement au niveau des signaux produits par le dispositif de réception de lumière réfléchie.

La compensation de l'influence de la lumière ambiante est réalisée avantageusement en effectuant les mesures suivant le principe de "détection synchrone", c'est-à-dire en effectuant des cycles de mesure au cours desquels la réflexion spéculaire et la réflexion diffuse sont mesurées quand le dispositif d'émission de de lumière fonctionne et quand le dispositif d'émission de lumière est à l'arrêt. Pour la commande du déroulement des mesures et l'exploitation des résultats, le dispositif de mesure utilise avantageusement des moyens de traitement numérique tels qu'un micro-ordinateur.

On notera encore que l'affichage de la brillance permet à l'opérateur de visualiser immédiatement la grandeur qu'il recherche et procure, en particulier, un moyen d'assistance pour un positionnement correct de la sonde.

La brillance obtenue est une grandeur relative élaborée à partir des mesures de la réflexion spéculaire et de la réflexion diffuse, par exemple la différence ou le quotient entre les valeurs de réflexion spéculaire et diffuse mesurées. La différence est préférée au quotient dans la mesure où elle introduit moins de distorsion d'échelle par rapport à l'impression visuelle de la brillance de la peau. Une échelle de brillance peut être définie à partir d'une mesure d'un mat de référence (unité 1) et d'un miroir étalonné (unité $10^n$, n étant un entier quelconque supérieur à 0).

L'invention sera mieux comprise à la lecture de la description faite ci-après, à titre indicatif mais non limitatif, en référence aux dessins annexés sur lesquels:

— la figure 1 est un schéma général d'un mode de réalisation d'un appareil de mesure de brillance selon l'invention,

— la figure 2 est une vue plus détaillée et en coupe de la sonde de l'appareil de la figure 1,

— la figure 3 illustre plus en détail la structure de l'émetteur du dispositif d'émission de l'appareil de la figure 1,

— la figure 4 est un schéma des circuits des dispositifs d'émission et de réception et du circuit d'interface de l'appareil de la figure 1,

— la figure 5 illustre la variation dans le temps de la tension de sortie du dispositif de réception au cours d'un cycle de mesure,

— les figures 6 et 7 sont des organigrammes relatifs aux opérations effectuées sous la commande des moyens de traitement numérique respectivement pour mesure et pour étalonnage.

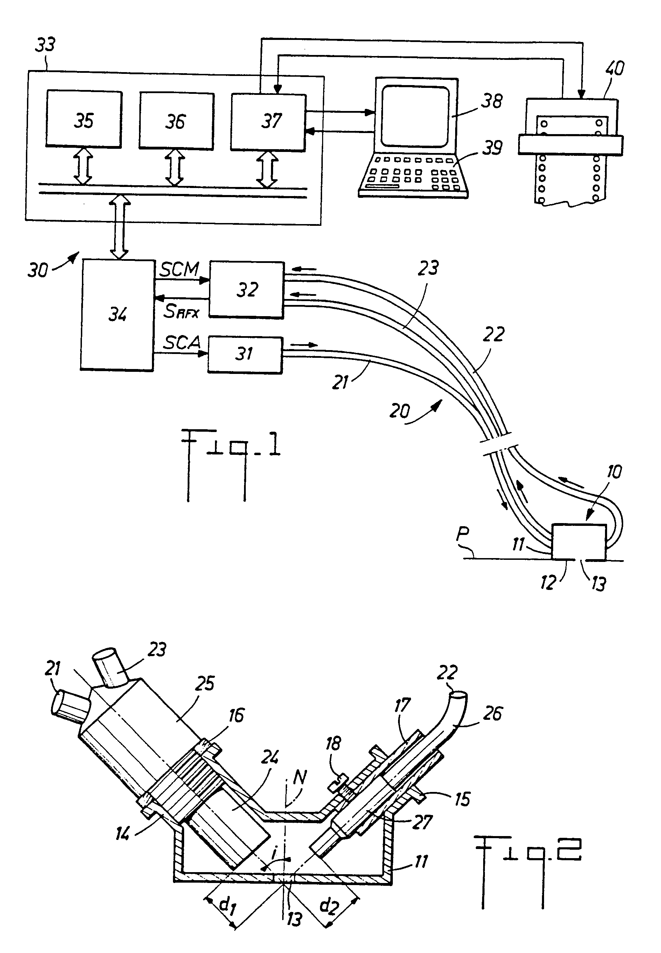

L'appareil illustré très schématiquement par la figure 1 comporte une sonde 10, un dispositif de mesure 30 et une liaison souple 20 à fibres optiques reliant le dispositif de mesure à la sonde 10.

La sonde 10 est destinée à être placée au contact de la peau P pour éclairer une partie à examiner de la surface de celle-ci.

La liaison 20 comprend trois voies optiques 21, 22, 23. La voie 21 est destinée à acheminer à la sonde la lumière produite par un dispositif d'émission de lumière 31 afin d'illuminer la partie de surface de peau à examiner. La voie 22 transmet à un dispositif de réception 32 la lumière réfléchie spéculairement (normalement) par la partie de surface examinée tandis que la voie 23 transmet au dispositif de réception 32 une partie de la lumière réfléchie de façon diffuse. Dans l'exemple illustré, la réflexion diffuse est mesurée dans la direction opposée à la direction d'incidence de la lumière sur la surface à examiner. Les voies 21 et 23 peuvent alors être réunies, au moins du côté de la sonde, dans un câble optique bidirectionnel.

Les dispositifs d'émission 31 et de réception 32 sont reliés à un dispositif de traitement et de commande 33 par l'intermédiaire d'un circuit d'interface 34.

Le dispositif 31 comprend des moyens de régulation de l'intensité lumineuse produite et est déclenché par des signaux de commande émis par le dispositif de traitement 33.

Le dispositif de réception 32 comprend des transducteurs photo-électriques pour élaborer des signaux électriques représentatifs de la réflexion normale et de la réflexion diffuse. Ces signaux sont transmis au dispositif de traitement 33 à travers le circuit d'interface 34, la transmission étant réalisée sous la commande de signaux produits par le dispositif de traitement.

De façon classique, le dispositif de traitement 33 comprend des circuits de mémoire 35, une unité arithmétique et logique 36 et des circuits d'interface 37 permettant la liaison avec un dispositif de visualisation 38, tel qu'un tube à rayons cathodiques, avec un clavier 39 et avec une imprimante 40. Le dispositif de traitement peut être constitué par tout microordinateur existant; il n'est donc pas nécessaire de le décrire ici plus en détail.

3

L'alimentation des différents circuits de l'appareil est assurée par des circuits d'alimentation (non représentés).

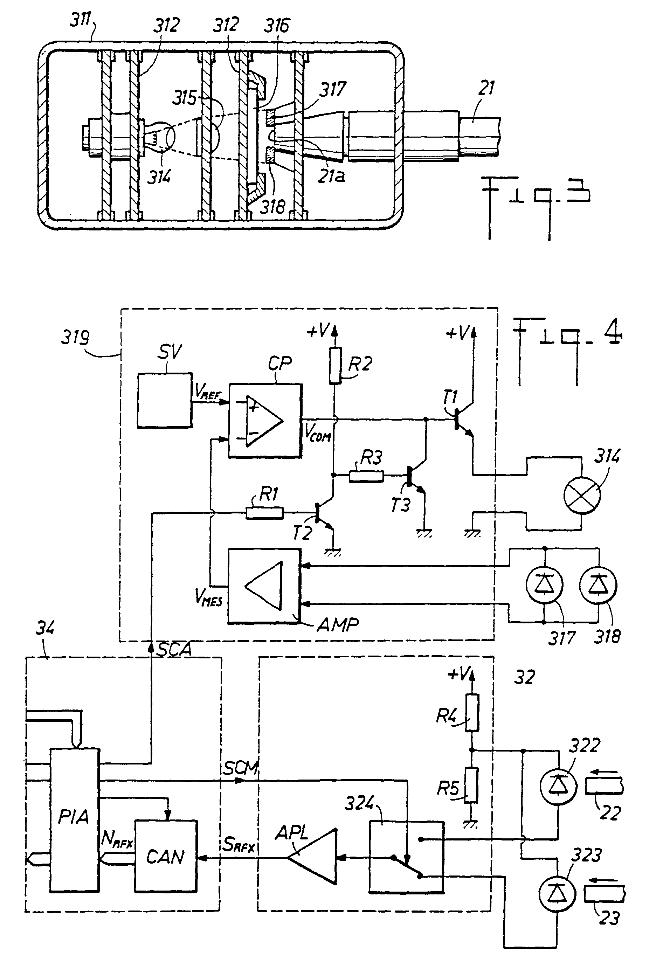

La figure 2 est une vue schématique en coupe montrant la sonde 10 de façon plus détaillée.

La sonde 10 comprend un boîtier 11 dont la face frontale 12 présente en son centre une ouverture 13 par exemple de forme circulaire. Le boîtier présente en outre deux parties de raccordement 14, 15 dans lesquelles sont fixées respectivement les extrémités des voies 21, 23 et l'extrémité de la voie 22. A leurs extrémités, les voies 21, 23 sont réunies en un câble optique bidirectionnel 24 muni d'un embout 25 vissé dans la partie de raccordement 14, tandis que le câble optique 26 formant la voie 22 est muni d'une bague 27 et est inséré dans un guide tubulaire 17 logé dans la partie de raccordement 15.

L'axe du câble optique 24, c'est-à-dire l'axe de la partie de raccordement 14 passe par le centre de l'ouverture 13 et est incliné par rapport à la normale N à la face frontale 12 d'un angle i correspondant à l'angle choisi pour l'incidence sous laquelle la partie de surface de peau à examiner est éclairée. Dans l'exemple illustré, l'angle d'incidence i est égal à environ 45°, mais une autre valeur pourrait être choisie. L'axe du câble optique 26, c'est-à-dire l'axe de la partie de raccordement 15 est symétrique de l'axe du câble 24 par rapport à la normale N passant par le centre de l'ouverture 13 puisque la voie 22 est destinée à recueillir la lumière réfléchie normalement.

Les câbles 24 et 26 sont fixés au boîtier 11 de sorte que les extrémités des fibres optiques qui les composent se trouvent à des distances prédéterminées d1 et d2 du centre de l'ouverture 13. Le réglage de la position de l'extrémité du câble 24 est assuré par interposition de cales 16 entre l'embout 25 et la partie de raccordement 14 tandis que l'extrémité du câble 26 est fixé dans la position voulue dans le guide 17 au moyen d'une vis pointeau 18 traversant la partie de raccordement 15 et s'appuyant sur la bague 27. A titre indicatif, les distances d1 et d2 sont d'environ 20 mm.

L'utilisation d'une liaison souple à fibres optiques dont les extrémités sont fixées à la sonde apporte plusieurs avantages. Ainsi, la sonde peut être de taille réduite, son encombrement étant déterminé par les moyens de raccordement des câbles optiques. De plus, la sonde ne comporte pas d'éléments d'optique tels que des lentilles qui requièrent une grande précision de positionnement.

La zone de mesure, déterminée par la taille de l'ouverture 13 peut alors être suffisamment petite pour permettre des mesures significatives sur une surface aussi peu rigide et uniforme que la peau. A titre indicatif, la superficie de la zone de mesure peut être de 10 à 50 mm², par exemple environ 25 mm². La miniaturisation de la sonde et sa liaison souple avec le reste de l'appareil permettent de surcroît une manipulation aisée pour faire des mesures sur différentes zones de la surface de la peau.

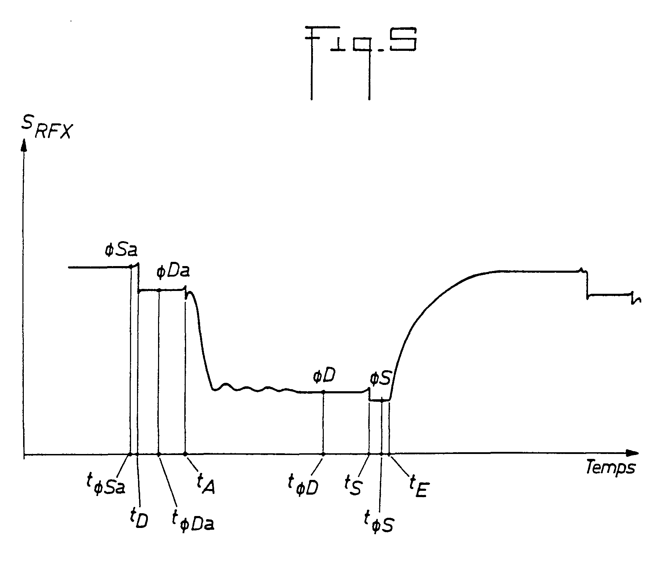

La figure 3 illustre schématiquement la structure de l'émetteur du dispositif d'émission de lumière 31.

Cet émetteur comprend un boîtier 311 auquel est raccordée l'extrémité de départ de la voie optique 21. Le boîtier 311 est muni de cloisons 312 servant de support aux différents éléments logés dans le boîtier. La source lumineuse est une lampe 314 à filament de tungstène. Le faisceau produit par la lampe est focalisé au moyen d'une lentille 315 afin d'obtenir un flux lumineux suffisant à l'entrée 21a de la voie optique 21. Un filtre infrarouge 316 peut être interposé entre la lampe 314 et l'entrée de la voie optique 21 afin de faire des mesures dans le domaine de la lumière visible dénuée d'infrarouge. Deux photodiodes 317, 318 sont placées des deux côtés de l'entrée de la voie optique 21 afin de pouvoir fournir des signaux représentatifs de l'intensité du flux à cette entrée. Les photodiodes 317 et 318 sont reliées à un circuit 319 de régulation de l'intensité lumineuse produite par la lampe 314.

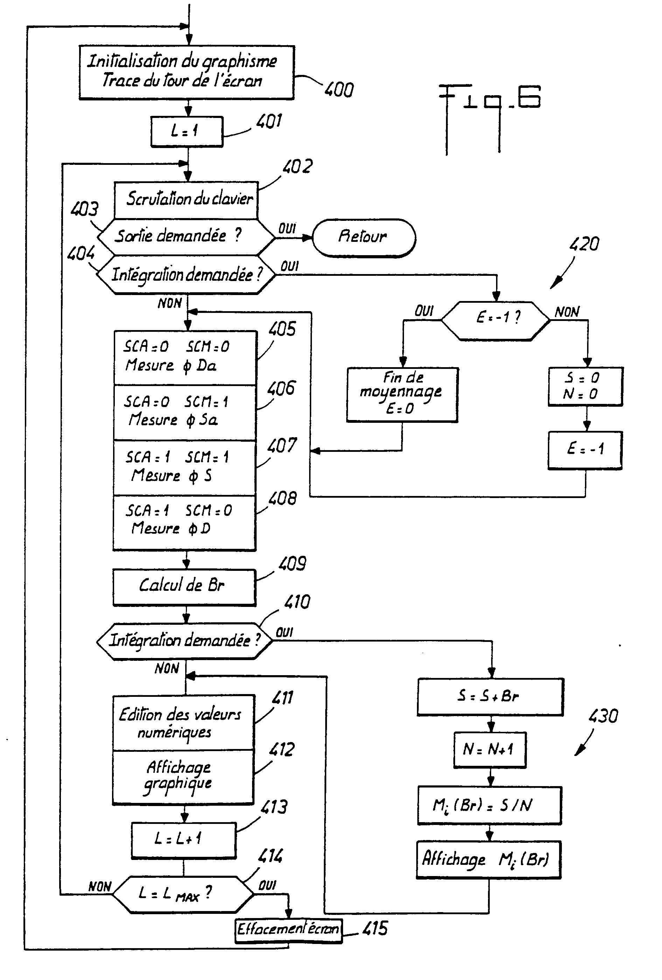

Le circuit de régulation 319 (figure 4) comporte une source de courant comprenant un transistor T1 dont le collecteur est au potentiel +V d'une source d'alimentation et dont l'émetteur est relié à une borne au potentiel de référence (masse) par l'intermédiaire de la lampe 314. Les photodiodes 317, 318 sont reliées à un circuit d'amplification AMP qui délivre une tension $V_{MES}$ représentant l'intensité réelle du flux lumineux appliqué à l'entrée de la voie 21. La tension $V_{MES}$ est comparée à une tension de référence $V_{REF}$, fournie par un générateur de tension ajustable SV; la comparaison est effectuée au moyen d'un circuit différentiel CP qui délivre une tension $V_{COM}$ fonction de la différence entre $V_{REF}$ et $V_{MES}$. La tension $V_{COM}$ est appliquée à la base de T1 et détermine le courant dans la lampe 314 de manière à ramener vers zéro l'écart entre les tensions $V_{REF}$ et $V_{MES}$.

Le circuit 319 reçoit un signal de commande d'allumage SCA appliqué par l'intermédiaire d'une résistance R1 à la base d'un transistor T2. L'émetteur de celui-ci est relié à la masse tandis que son collecteur est relié, d'une part, à la source de tension +V par une résistance R2, et, d'autre part à la base d'un transistor T3 par une résistance R3. Ce dernier a son circuit collecteur-émetteur branché entre la base de T1 et la masse. Lorsque le signal de commande d'allumage est à un niveau inférieur au seuil de déclenchement du transistor T2 (SCA=0, ou niveau logique bas), le transistor T2 est bloqué, mais le transistor T3 est passant, portant la base de T1 à la masse; la lampe 314 est éteinte. Lorsque le signal de commande d'allumage dépasse le seuil de déclenchement de T2 (SCA=1, ou niveau logique haut), le transistor T2 conduit, ce qui maintient T3 à l'état bloqué et la lampe 314 est allumée, l'intensité du courant la traversant étant déterminée pr $V_{COM}$.

La figure 4 montre également le circuit du dispositif de réception 32.

Deux photodiodes 322, 323 reçoivent les flux lumineux transmis respectivement par les voies optiques 22, 23. Les diodes 322, 323 sont des photodiodes au silicium branchées en inverse avec leurs cathodes reliées au point milieu d'un diviseur de tension formé par deux résistances R4, R5 connectées en série entre la masse et une borne au potentiel +V. Les diodes 322, 323 produisent ainsi un courant sensiblement proportionnel au flux lumineux capté. Les anodes des diodes 322, 323 sont reliées à deux contacts d'entrée d'un commutateur analogique 324 dont le contact de sortie est relié à l'entrée d'un amplificateur logarithmique APL qui fournit un signal analogique $S_{RFX}$ représentatif de la réflexion spéculaire ou de la réflexion diffuse selon l'état du commutateur 324. L'utilisation d'un amplificateur logarithmique procure une grande dynamique. De plus, l'oeil humain constituant un récepteur de type logarithmique, l'appareil de mesure permet de se rapprocher de l'impression visuelle qu'il est destiné à quantifier.

Le dispositif de réception reçoit un signal de commande de commutation SCM destiné à commander l'état du commutateur. Par exemple, lorsque le signal SCM a un niveau logique haut (SCM=1), le commutateur 234 relie la photodiode 232 à l'amplificateur APL pour mesurer la réflexion spéculaire tandis que, lorsque le signal SCM a un niveau logique bas (SCM=0), le commutateur 234 relie la photodiode 233 à l'amplificateur APL pour mesurer la réflexion diffuse.

Le circuit d'interface 34 comprend un convertisseur analogique-numérique CAN qui reçoit le signal $S_{RFX}$ pour le convertir sous forme d'un mot numérique $N_{RFX}$ de n bits. Un circuit de liaison PIA ("parallel interface adapter") est interposé entre le convertisseur CAN et le micro-ordinateur 33. Le circuit PIA transmet également les signaux SCA et SCM ainsi que les signaux de commande du convertisseur CAN. Le circuit PIA est commandé de façon connue en soi par des signaux de commande produits par le micro-ordinateur.

Les dispositifs d'émission et de réception sont commandés pour réaliser une mesure de brillance à partir des valeurs de réflexion spéculaire et de réflexion diffuse; dans le cas envisagé ici, la valeur élaborée est représentative de la différence entre les intensités de réflexion spéculaire et de réflexion diffuse. De plus, pour tenir compte de l'influence de la lumière ambiante, la réflexion est mesurée suivant un principe de "détection synchrone", c'est-à-dire en commandant alternativement l'allumage et l'extinction de la source lumineuse.

Le flux lumineux $\Phi S$ acheminé par la voie 22 (réflexion spéculaire) est composé du flux $\Phi Sp$ effectivement réfléchi par la peau, du flux $\Phi Sa$ provenant de l'extérieur (lumière ambiante) et des fuites des détecteurs et du flux $\Phi Ss$ renvoyé par le boîtier de la sonde. De même, le flux lumineux $\Phi D$ acheminé par la voie 23 (réflexion diffuse) comprend les composantes $\Phi Dp$, $\Phi Da$ et $\Phi Ds$).

Au cours d'un cycle de mesure, les flux $\Phi Da$, $\Phi Sa$ sont successivement mesurés par action sur le commutateur 324, la lampe étant éteinte, puis, après allumage de la lampe, les flux $\Phi S$ et $\Phi D$ sont mesurés successivement en agissant sur le commutateur 324.

La brillance recherchée est égale à:

$$Br=\Phi Sp-\Phi DP=(\Phi S-\Phi Sa-\Phi Ss)-(\Phi D-\Phi Da-\Phi Ds)/K,$$

K étant un facteur correctif tenant compte de la géométrie de la sonde et des voies optiques 22, 23 puisque la brillance est évaluée par différence entre intensités des réflexions spéculaire et diffuse, et non différence entre flux.

Les quantités $\Phi Ss$, $\Phi Ds$ et K sont déterminées par étalonnage. En plaçant la sonde devant un piège à lumière (à la place de la peau), on mesure $\Phi Sa+\Phi Ss$ et $\Phi Da+\Phi Ds$, lorsque la lampe est allumée, et on mesure $\Phi Sa$ et $\Phi Da$, lorsque la lampe est éteinte, d'où l'on déduit $\Phi Ss$ et $\Phi Ds$. La valeur de K est ensuite déterminée en plaçant la sonde devant une surface mate servant de référence de brillance nulle (Br=0), en mesurant $\Phi D$, $\Phi S$, $\Phi Da$ et $\Phi Ss$, et en calculant:

$$K=(\Phi D-\Phi Da-\Phi Ds)/(\Phi S-\Phi Sa-\Phi Ss).$$

On détermine encore un facteur d'échelle FE en plaçant la sonde devant une surface réfléchissante de référence telle qu'un miroir étalonné à 80% de réflexion, la brillance étant alors arbitrairement fixée à une valeur prédéterminée BrM (par exemple 1000). Après mesure de $\Phi D$, $\Phi S$, $\Phi Da$ et $\Phi Ss$, le facteur FE est déterminé par la division de BrM par la quantité:

$$(\Phi S-\Phi Sa-\Phi Ss)-(\Phi D-\Phi Da-\Phi Ds)/K.$$

Les valeurs de $\Phi Ss$, $\Phi Ds$, K et FE déterminés par étalonnage sont mémorisées dans les circuits de mémoire 35 du microordinateur.

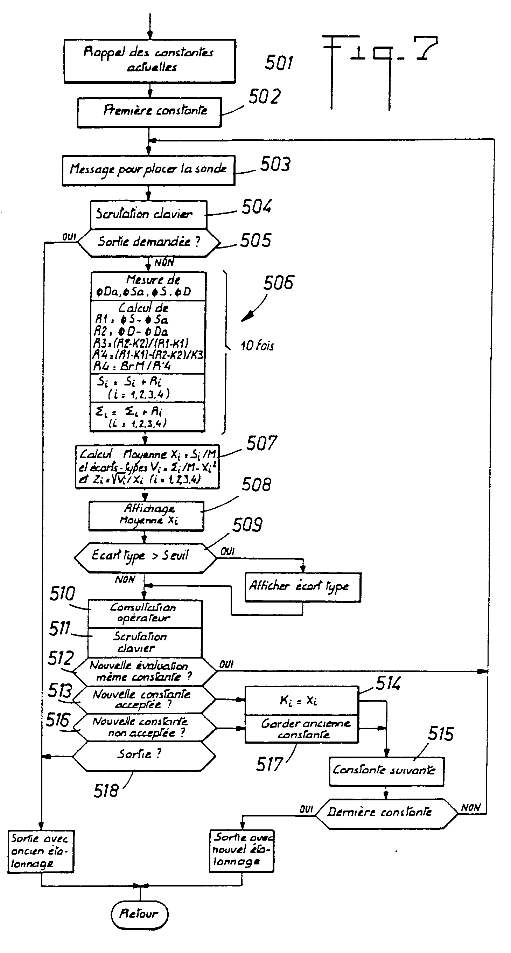

La figure 5 représente la variation dans le temps de la tension $S_{RFX}$ en sortie de l'amplificateur logarithmique APL. Les temps $t_{\phi Sa}$, $t_{\phi Da}$, $t_{\phi D}$, $t_{\phi S}$ correspondent aux instants de prélèvement des grandeurs $\Phi Sa$, $\Phi Da$, $\Phi D$ et $\Phi S$. Les temps $t_A$ et $t_E$ correspondent aux instants d'allumage et d'extinction de la lampe, tandis que les temps $t_S$ et $t_D$ correspondent aux instants de commutation du commutateur 234 respectivement vers la photodiode 232 (réflexion spéculaire) et vers la photodiode 233 (réflexion diffuse).

Les cycles de mesure successifs sont réalisés sous la commande du micro-ordinateur. La durée d'un cycle peut être inférieure à 1 s, par exemple environ 0,7 s, cette durée étant notamment fonction des temps nécessaires à la stabilisation de la lampe à l'allumage et à l'extinction. Les valeurs de la brillance Br calculées au cours de cycles successifs de mesure sont affichées sous forme de positions successives d'un curseur sur l'écran du tube 38. L'opérateur peut ainsi corriger un éventuel mauvais placement de la sonde en observant les variations de position en ordonnée du curseur lorsqu'il déplace légèrement la sonde.

5

L'affichage instantané de la valeur calculée de la brillance apporte ainsi une assistance au positionnement de la sonde. La valeur de brillance finalement retenue peut être une valeur moyenne élaborée à partir des résultats d'un nombre prédéterminé de cycles de mesure. Cette valeur finale peut être éditée sur l'imprimante 40 et est affichée à l'écran. La valeur de brillance obtenue est enregistrée dans un fichier informatique comportant des informations complémentaires relatives au patient dont la peau est à examiner, à la date de l'examen et, éventuellement, à des conditions particulières d'examen. Les informations enregistrées peuvent être éditées sur papier au moyen de l'imprimante, à la demande de l'opérateur.

Le programme principal comprenant les opérations d'initialisation du système et les sous-programmes d'enregistremen ten fichier et de lecture du fichier sont des phases non spécifiques de l'application envisagée; elles ne seront donc pas décrites en détail ici.

Les opérations de mesure et d'étalonnage mettent en oeuvre les programmes dont les organigrammes sont représentés aux figures 6 et 7.

En ce qui concerne la mesure, les phases suivantes sont réalisées:

— initialisation du graphisme et tracé du tour de l'écran en vue de l'affichage des résultats de mesure sous forme d'une courbe représentative de la variation de la brillance (phase 400);

— placement du curseur à l'abscisse L=1 sur l'écran (phase 401);

— scrutation du clavier (phase 402);

— si, par action de l'opérateur sur le clavier, la sortie du sous-programme est demandée (test 403), retour au programme principal;

— si, par action de l'opérateur sur le clavier, une intégration est demandée sur les valeurs de brillance obtenues au cours de cycles successifs de mesure (test 404), un sous-programme (420) est appelé au cours duquel un test est effectué sur le positionnement d'un indicateur de moyennage (E=−1 ?) pour, dans l'affirmative, arriver en fin de moyennage, ramener l'indicateur E à zéro et retourner au programme et, dans le négative, mettre la somme S et le paramètre N à zéro, positionner E à −1 et retourner au programme;

— mesure du flux $\Phi Da$, les signaux SCA et SCM étant positionnés à 0 et lecture de la valeur numérique correspondante (phase 405);

— commutation de la voie 23 à la voie 22 en positionnant SCM à 1, mesure du flux $\Phi Sa$ et lecture de la valeur numérique correspondante (phase 406);

— allumage de la lampe par positionnement de SCA à 1, mesure du flux $\Phi S$ et lecture de la valeur numérique correspondante (phase 407);

— commutation de la voie 22 à la voie 23 par positionnement de SCM à 0, mesure de $\Phi D$ et lecture de la valeur numérique correspondante (phase 408);

— calcul de Br à partir des valeurs lues de $\Phi Da$, $\Phi Sa$, $\Phi S$ et $\Phi D$ et des valeurs pré-enregistrées de $\Phi Ss$, $\Phi Ds$, K et FE (phase 409);

— si une intégration est demandée (test 410), appel d'un sous-programme 430 de sommation comprenant la mise à jour de la somme S (S=S+Br), l'incrémentation de N (N=N+1), le calcul d'une "moyenne instantanée" de brillance $M_i(Br)=S/N$, la commande de l'affichage à l'écran de la valeur numérique de $M_i(Br)$ et le retour au programme;

— édition des valeurs numériques de Br ou, éventuellement de $M_i(Br)$ (phase 411);

— affichage graphique de la valeur numérique de Br par commande de l'ordonnée du curseur à l'écran (phase 412);

— incrémentation de l'abscisse du curseur à l'écran: L=L+1 (phase 413)

— si la valeur de L est égale à l'abscisse maximale possible $L_{MAX}$ (test 414), effacement de l'écran (phase 415) et retour à l'initialisation du graphisme sinon retour à la phase 402.

En ce qui concerne l'étalonnage, les phases suivantes sont réalisées:

— rappel des constantes actuelles (phase 501);

— passage à la première constante (phase 502);

— affichage à l'écran d'un message (phase 503) pour placer la sonde devant la surface correspondant à la constante à déterminer (piège à lumière, surface mate de référence, miroir de référence);

— scrutation du clavier (phase 504);

— si, par action de l'opérateur sur le clavier, la sortie du sous-programme est demandée (test 505), retour au programme principal sans modifier l'étalonnage;

— pour chacune des constantes à déterminer K1 à K4 (K1=$\Phi Ss$, K2=$\Phi Ds$, K3=K et K4=FE), réalisation de M cycles successifs, par exemple 10 cycles de mesure (phase 506) comprenant chacun:

· la mesure des flux $\Phi Da$, $\Phi Sa$, $\Phi S$ et $\Phi D$ (phases 405 à 408 du programme de mesure ci-dessus);

· le calcul des quantités
R1=$\Phi S$−$\Phi Sa$,
R2=$\Phi D$−$\Phi Da$, R3=(R2−K2)/(R1−K1),
R'4=(R1−K1)−(R2−K2)/K3 et
R4=BrM/R'4;

· la mise à jour de la somme Si par:
Si=Si+Ri (i=1, 2, 3 ou 4); et

· la mise à jour de la somme ΣI par

$\Sigma i = \Sigma i + Ri^2$ (i=1, 2, 3 ou 4);

— calculs de moyenne et écart-type pour chaque constante (phase 507), c'est-à-dire la moyenne Xi=Si/M, l'écart-type Vi=$\Sigma i/M-Xi^2$ et l'écart-type réduit Zi=$\sqrt{Vi}/Xi$: (i=1, 2, 3, ou 4);

— affichage de la valeur moyenne X, calculée (phase 508);

— si l'écart-type réduit est supérieur à un seuil prédéterminé (test 509), il est affiché à l'écran, sinon passage direct à la phase suivante;

— consultation de l'opérateur (phase 510)

— scrutation du clavier (phase 511)

— si, par action de l'opérateur sur le clavier, une nouvelle évaluation de la même constante est demandée (test 512), retour à la phase 503;

— si, par action de l'opérateur sur le clavier, la nouvelle constante est conservée (test 513), alors Ki=Xi (phase 514) et passage à la constante suivante (phase 515);

— si, par action de l'opérateur sur le clavier, la valeur Xi n'est pas acceptée (test 516), alors la valeur actuelle de la constante est conservée (phase 517) et passage à la constante suivante (phase 515);

— si, par action sur le clavier, la sortie est demandée (test 518), retour au programme principal sans modifier l'étalonnage;

— au passage à la constante suivante (phase 515) et si les quatre constantes n'ont pas encore été évaluées (test 519), retour à la phase 503;

— si toutes les constantes ont été évaluées, sortie avec modification de l'étalonnage et retour au programme principal.

Des essais ont été effectués avec un appareil de mesure tel que décrit ci-avant en utilisant une échelle de brillance Br allant de 0 pour la surface mate de référence à 1 000 pour la surface réfléchissante de référence (miroir à 80% de réflexion).

Les mesures effectuées sur 34 personnes ont donné des valeurs de brillance dans une plage allant de 8 à 12,5 pour l'avant-bras et de 6 à 13,9 pour le front.

Dans le cas de personnes (7 cas) dont la peau parait grasse à l'examen visuel, la valeur moyenne de brillance mesurée sur le front a été de 11,7 à comparer avec la valeur moyenne générale de 9,56 obtenue à partir de mesures effectuées dans 32 cas sans sélection. Par ailleurs, des mesures effectuées sur cinq sujets ont montré un écart de 5,4 entre les valeurs moyennes de brillance obtenues avant et après application de vaseline sur l'avant-bras.

Ces résultats démontrent la corrélation effective entre l'aspect visuel de brillance et la mesure réalisée, justifiant ainsi l'utilisation de l'appareil de mesure selon l'invention comme moyen "objectif" de quantification de la brillance de la peau.

**Revendications**

1. Appareil de mesure de la brillance de la peau comprenant

— une sonde (10) comprenant un boîtier (11) dont une face destinée à être amenée au contact de la peau présente une ouverture (13), caractérisé en ce qu'il comporte:

— une liaison souple (20) à fibres optiques comprenant au moins trois conducteurs optiques (21, 22, 23) qui, à une première extrémité, sont fixés dans le boîtier de la sonde en étant dirigés vers l'ouverture (13) de celle-ci, un premier et un deuxième conducteurs (21, 22) ayant leurs extrémiés orientées suivant une première et une deuxième directions symétriques l'une de l'autre par rapport à l'axe (N) de l'ouverture, et un troisième conducteur (23) ayant son extrémité orientée suivant une direction autre que ladite seconde direction,

— un dispositif de mesure comprenant: un dispositif d'emission de lumière (31) couplé optiquement à l'autre extrémité du premier conducteur (21); un dispositif de réception de lumière (32) couplé optiquement à l'autre extrémité du deuxième conducteur (22) pour fournir un premier signal représentatif de la réflexion spéculaire et à l'autre extrémité du troisième conducteur (23) pour fournir un second signal représentatif d'une partie de la réflexion diffuse; et des moyens de traitement (33, 34) connectés aux dispositifs d'émission et de réception de lumière, munis de moyens de correction pour s'affranchir de variations de l'intensité de la lumière émise et de l'influence de la lumière ambiante, et destinés à fournir un signal relatif de brillance (Br) à partir des valeurs mesurées de réflexion spéculaire et de réflexion diffuse, et

— un dispositif d'affichage (38) recevant le signal de brillance pour indiquer l'amplitude de ce signal.

2. Appareil selon la revendication 1, caractérisé en ce que le dispositif d'émission de lumière (31) comprend au moins un détecteur photo-électrique (317, 318) destiné à fournir un signal représentatif de l'intensité de l'éclairement produit par une source de lumière (314) et un circuit de régulation (319) relié au détecteur et à la source de lumière pour commander celle-ci de manière à maintenir sensiblement constante l'intensité de l'éclairement qu'elle produit.

3. Appareil selon l'une quelconque des revendications 1 et 2, caractérisé en ce que les dispositifs (31, 32) d'émission et de réception de lumière sont commandés au moyen de signaux (SCA, SCM) produits par les moyens de traitement (33) pour réaliser des cycles de mesure successifs comprenant chacun deux mesures de la réflexion spéculaire ($\Phi S$, $\Phi Sa$), respectivement lorsque le dispositif d'émission (31) fonctionne et lorsqu'il est à l'arrêt, et deux mesures de la réflexion diffuse ($\Phi D$, $\Phi Da$), respectivement lorsque le dispositif d'émission (31) fonctionne et lorsqu'il est à l'arrêt.

# EP 0 198 759 B1

4. Appareil selon la revendication 3, caractérisé en ce que les moyens de traitement (33) comportent des moyens de calcul pour corriger les valeurs des réflexions spéculaire et diffuse (ΦS, ΦD) mesurées lorsque le dispositif d'émission (31) fonctionne, de l'influence de la lumière ambiante représentée par les valeurs des réflexions spéculaire et diffuse (ΦSa, ΦDa) mesurées lorsque le dispositif d'émission (31) est à l'arrêt.

5. Appareil selon l'une quelconque des revendications 3 et 4, caractérisé en ce que les moyens de calcul sont en outre agencés pour corriger les valeurs de réflexions spéculaire et diffuse (ΦS, ΦD) mesurées lorsque le dispositif d'émission (31) fonctionne, de l'influence de la lumière parasite réfléchie par la sonde représentée par des valeurs de réflexions parasites spéculaire et diffuse (ΦSs, ΦDs) prédéterminées par étalonnage.

6. Appareil selon l'une quelconque des revendications 1 à 5, caractérisé en ce que les moyens de traitement (33) sont agencés pour élaborer une grandeur de brillance par différence entre les intensités des rayonnements réfléchis par la peau de façon spéculaire et de façon diffuse.

7. Appareil selon l'une quelconque des revendications 1 à 6, caractérisé en ce que: le dispositif de réception comporte un premier et un deuxième récepteurs photo-électriques (322, 323) couplés respectivement au deuxième et au troisième conducteurs optiques (22, 23) et un commutateur (234) inséré entre les récepteurs et un convertisseur analogique-numérique (CAN) pour relier ce dernier à l'un ou l'autre des récepteurs sous la commande d'un signal (SCM) produit par les moyens de traitement.

8. Appareil selon l'une quelconque des revendications 1 à 7, caractérisé en ce que le dispositif de réception comporte un amplificateur logarithmique (APL) destiné à amplifier les signaux fournis par des récepteurs photo-électriques (322, 323) couplés respectivement au deuxième et au troisième conducteurs optiques (22, 33).

9. Appareil selon l'une quelconque des revendications 1 à 8, caractérisé en ce que le dispositif d'émission de lumière (31) comprend une souce lumineuse (314) et un moyen interrupteur (T2) connecté à la source lumineuse pour provoquer l'allumage ou l'extinction de celle-ci sous la commande d'un signal (SCA) produit par les moyens de traitement.

**Patentansprüche**

1. Vorrichtung zur Messung der Helligkeit der Haut mit
— einer Sonde (10), die ein Gehäuse (11) aufweist, wovon die Seite, die dazu bestimmt ist, mit der Haut in Kontakt gebracht zu werden, eine Öffnung (13) aufweist, gekennzeichnet durch
— eine biegsame Verbindung (20) aus optischen Fasern, die aus zumindest drei optischen Leitern (21, 22, 23) besteht, die mit einem ersten Ende an dem Gehäuse der Sonde befestigt sind und auf die Öffnung (13) desselben hin gerichtet sind, wobei ein erster und ein zweiter Leiter (21, 22) ihre Enden in in bezug auf die Achse (N) der Öffnung paarweise symmetrischen Richtungen ausgerichtet haben und wobei ein dritter Leiter (23) sein Ende längs einer Richtung, die anders als die zweite Richtung ist, ausgerichtet hat,
— eine Meßvorrichtung mit: einer Vorrichtung zur Lichtemission (31), die optisch mit dem anderen Ende des ersten Leiters (21) gekoppelt ist; einer Vorrichtung zum Empfangen des Lichtes (32), die optisch mit dem anderen Ende des zweiten Leiters (22) gekoppelt ist, um ein erstes Signal bereitzustellen, das der gerichteten Reflexion entspricht und die außerdem mit dem anderen Ende des dritten Leiters (23) verbunden ist, um ein zweites Signal bereitzustellen, das einem Teil der diffusen Reflexion entspricht; und mit verarbeitenden Mitteln (33, 34), die mit den Vorrichtungen zur Lichtemission und zum Empfangen des Lichtes verbunden sind und die mit Korrekturmitteln versehen sind, um Veränderungen der Intensität des ausgesandten Lichtes und des Einflusses des Umgebungslichtes auszugleichen und die dazu bestimmt sind, ausgehend von den gemessenen Werten der diffusen Reflexion und der direkten Reflexion, ein Signal in bezug auf die Helligkeit (Br) bereitzustellen, und
— eine Anzeigevorrichtung (38), die das Helligkeitssignal empfängt, um die Amplitude dieses Signals anzuzeigen.

2. Vorrichtung nach Anspruch 1, dadurch gekennzeichnet, daß die Vorrichtung zur Lichtemission (31) zumindest einen photoelektrischen Dektor (317, 318) aufweist, der zur Bereitstellung eines Signales vorgesehen ist, das der Intensität und der Beleuchtungsstärke, die von einer Lichtquelle (314) erzeugt wird, entspricht, sowie einen Regelkreis (319), der mit dem Detektor und der Lichtquelle verbunden ist, um selbige so zu steuern, daß die von ihr erzeugte Beleuchtungsstärke annähernd konstant gehalten wird.

3. Vorrichtung nach einem der Ansprüche 1 und 2, dadurch gekennzeichnet, daß die Vorrichtungen (31, 32) zur Emission und zum Empfangen des Lichtes mittels Signalen (SCA, SCM) gesteuert werden, die von den verarbeitenden Mitteln (33) erzeugt werden, um aufeinanderfolgende Meßzyklen zu erzeugen, die alle zwei Messungen der direkten Reflexion (ΦS, ΦSa) umfassen, jeweils wenn die Vorrichtung zur Emission (31) arbeitet bzw. wenn sie nicht arbeitet, und zwei Messungen der diffusen Reflexion (ΦD, ΦDa), jeweils wenn die Vorrichtung zur Emission (31) arbeitet bzw. wenn sie nicht arbeitet.

4. Vorrichtung nach Anspruch 3, dadurch gekennzeichnet, daß die verarbeitenden Mittel (33) Rechenmittel zum Korrigieren der Werte der direkten und der diffusen Reflexion (ΦS, ΦD) aufweisen, die gemessen werden, wenn die Vorrichtung zur Emission (31) arbeitet, wobei den Einfluß des Umgebungslichtes die Werte der direkten und der diffusen Reflexion darstellen, (ΦSa, ΦDa), die gemessen wurden, wenn die Vorrichtung zur Emission (31) nicht arbeitet.

8

5. Vorrichtung nach einem der Ansprüche 3 und 4, dadurch gekennzeichnet, daß die Rechenmittel außerdem ausgelegt sind, die Werte der direkten und der diffusen Reflexion (ΦS, ΦD), die gemessen werden, wenn die Vorrichtung zur Emission (31) arbeitet, um den Einfluß des parasitären Lichtes zu korrigieren, das von der Sonde reflektiert wird, und der durch die Werte der direkten und diffusen parasitären Reflexion (ΦSs, ΦDs) dargestellt wird und der durch Eichung vorbestimmt sind.

6. Vorrichtung nach einem der Ansprüche 1 bis 5, dadurch gekennzeichnet, daß die verarbeitenden Mittel (33) dazu ausgelegt sind, die Größe der Helligkeit durch Differenzbildung zwischen den von der Haut reflektierten direkten und diffusen Strahlungsintensitäten zu ermitteln.

7. Vorrichtung nach einem der Ansprüche 1 bis 6, dadurch gekennzeichnet, daß das empfangende Mittel einen ersten und einen zweiten photoelektrischen Rezeptor (322, 323) umfaßt, die jeweils mit dem zweiten und dem dritten optischen Leiter (22, 23) verbunden sind, sowie einen Umschalter (234), der zwischen den Rezeptoren und einem Analog-Digital-Wandler (CAN) eingesetzt ist, um letzteren mit dem einem oder anderen der Rezeptoren abhängig von einem Signal (SCM), das von den verarbeitenden Mitteln erzeugt wird, zu verbinden.

8. Vorrichtung nach einem der Ansprüche 1 bis 7, dadurch gekennzeichnet, daß die empfangenden Mittel einen logarithmischen Verstärker (APL) aufweisen, der zur Verstärkung der Signale vorgesehen ist, die von den photoelektrischen Rezeptoren (322, 323) bereitgestellt werden, die jeweils mit dem zweiten und dem dritten optischen Leiter (22, 23) verbunden sind.

9. Vorrichtung nach einem der Ansprüche 1 bis 8, dadurch gekennzeichnet, daß die Vorrichtung zur Emission des Lichtes (31) eine leuchtende Quelle (314) und ein unterbrechendes Mittel (T2) umfaßt, das mit der leuchtenden Quelle verbunden ist, um die Beleuchtung oder Löschung derselben in Abhängigkeit von einem von den verarbeitenden Mitteln bereitgestellten Signal (SCA) zu bewirken.

## Claims

1. A skin reflectance measuring apparatus comprising:
a probe (10) comprising a casing (11) of which one face, which will be in contact with the skin, is provided with an aperture (13), characterized in that it comprises:
— a flexible connection (20) in optical fibers, comprising at least three optical conductors (21, 22, 23) which, at a first end, are secured in the casing of the probe such as to face the aperture (13) thereof, the first and second conductors (21, 22) having their first end portions directed respectively in a first direction and a second direction both directions being symmetrical to each other with respect to the axis (N) of the aperture, while the third conductor (23) has its first end portion directed in another direction than said second direction,
— a measuring device comprising: light emitting means (31) optically coupled to the second end of the first conductor (21); light receiving means (32) optically coupled to the other end of the second conductor (22) to produce a first signal representing the specular reflection, and to the other end of the third conductor (23) to produce a second signal representing part of the diffuse reflection; and processing means (33, 34) connected to the light emitting and receiving means, and provided with correcting means to compensate for variations in the emitted light and for the influence of ambient light, said correcting means producing a relative reflectance signal (Br) from the measured values of specular reflection and diffuse reflection, and
— a display device (38) receiving the reflectance signal to indicate the amplitude of said signal.

2. Apparatus according to claim 1, characterized in that the light-emitting means (31) comprises at least one photo-electric detector (317, 318) designed to supply a signal representing the intensity of the light produced by a light source (314) and a regulation circuit (319) connected to said detector and said light source in order to control the latter so as to keep the intensity of the light produced by the latter substantially constant.

3. Apparatus according to any one of claims 1 and 2, characterized in that the light emitting and receiving means (31, 32) are controlled by signals (SCA, SCM) produced by the processing means (33) in order to perform successive cycles of measurement each one including two measurements of the specular reflection (ΦS, ΦSa) respectively when the light emitting means (31) is operative and when it is inoperative, and two measurements of the diffuse reflection (ΦD, ΦDa), respectively when the light emitting means (31) is operative and when it is inoperative.

4. Apparatus according to claim 3, characterized in that the processing means (33) comprise calculation means to compensate the values of specular and diffuse reflection (ΦS, ΦD) measured when the light emitting means (31) is operative, for the influence of ambient light represented by the values of specular and diffuse reflections (ΦSa, ΦDa) measured when the light emitting means (31) is inoperative.

5. Apparatus according to any one of claims 3 and 4, characterized in that said calculation means are also adapted to compensate the values of specular and diffuse reflections (ΦS, ΦD) measured when the light emitting means (31) operates, for the influence of the stray light reflected by the probe and represented by values of specular and diffuse stray reflections (ΦSs, ΦDs) predetermined by calibration.

6. Apparatus according to any one of claims 1 to 5, characterized in that said processing means (33) are adapted to work out a reflectance quantity by difference between the intensities of the light reflected by the skin in specular and in diffuse manner.

7. Apparatus according to any one of claims 1 to 6, characterized in that the light receiving means comprises first and second photo-electric receivers (322, 323) respectively coupled to the second and third optical conductors (22, 23) and a switch (234) inserted between said receivers, and a digital-to-analog converter (CAN), for connecting said converter with either one of the receivers under the control of a signal (SCM) produced by said processing means.

8. Apparatus according to any one of claims 1 to 7, characterized in that the light receiving means comprises a logarithmic amplifier (APL) designed to amplify the signals issued from photo-electric receivers (322, 325) respectively coupled to said second and third optical conductors (22, 23).

9. Apparatus according to any one of claims 1 to 8, characterized in that the light emitting means (31) comprises a light source (314) and switching means (T2) connected to the light source for switching said source on or off under the control of a signal (SCA) produced by said processing means.

Fig.1

Fig.2

EP 0 198 759 B1

Fig.3

Fig.4

2

Fig.5

Fig.6

Initialisation du graphisme
Trace du tour de l'écran — 400

L = 1 — 401

Scrutation du clavier — 402

403 — Sortie demandée ?  OUI → Retour

404 — Intégration demandée ?  OUI

420

NON

$E = -1$ ?  OUI → Fin de moyennage $E = 0$

NON → $S = 0$ $N = 0$

$E = -1$

SCA = 0   SCM = 0
Mesure $\phi$ Da — 405

SCA = 0   SCM = 1
Mesure $\phi$ Sa — 406

SCA = 1   SCM = 1
Mesure $\phi$ S — 407

SCA = 1   SCM = 0
Mesure $\phi$ D — 408

Calcul de Br — 409

Intégration demandée ? — 410  OUI

NON

Edition des valeurs numériques — 411

Affichage graphique — 412

L = L + 1 — 413

NON — $L = L_{MAX}$ ?  OUI — 414

$S = S + Br$

$N = N + 1$ — 430

$M_i (Br) = S / N$

Affichage $M_i (Br)$

Effacement écran — 415

Fig. 7

Rappel des constantes actuelles — 501

Première constante — 502

Message pour placer la sonde — 503

Scrutation clavier — 504

Sortie demandée ? — 505 — OUI

NON

Mesure de
$\phi Da, \phi Sa, \phi S, \phi D$

Calcul de
$R1 : \phi S - \phi Sa$
$R2 : \phi D - \phi Da$
$R3 : (R2-K2)/(R1-K1)$
$R'4 : (R1-K1)-(R2-K2)/K3$
$R4 : BrM/R'4$
$S_i : S_i + R_i$
$(i : 1,2,3,4)$
$\Sigma_i : \Sigma_i + R_i$
$(i : 1,2,3,4)$
— 506 — 10 fois

Calcul Moyenne $X_i : S_i/M$
et écarts-types $V_i : \Sigma_i/M - X_i^2$
et $Z_i : \sqrt{V_i}/X_i$ $(i : 1,2,3,4)$ — 507

Affichage Moyenne $X_i$ — 508

Écart Type > Seuil — 509 — OUI

NON

Afficher écart type

510 — Consultation opérateur

511 — Scrutation clavier

512 — Nouvelle évaluation même constante ? — OUI

513 — Nouvelle constante acceptée ? — $K_i = X_i$ — 514

516 — Nouvelle constante non acceptée ? — Garder ancienne constante

Sortie ? — 518

517

Constante suivante — 515

Sortie avec ancien étalonnage

Sortie avec nouvel étalonnage

OUI — Dernière constante — NON

Retour